# EUROPEAN PATENT SPECIFICATION

(11) **EP 3 639 800 B1**
(45) Date of publication and mention of the grant of the patent: **07.06.2023**
(21) Application number: 18818221.6
(22) Date of filing: 11.05.2018
(51) Int. Cl.: A61F 13/49, A61F 13/496

(54) **PULL-UP DISPOSABLE DIAPER**
ÜBERZIEHBARE EINWEGWINDEL
COUCHE-CULOTTE D'APPRENTISSAGE JETABLE

(30) Priority: 14.06.2017 JP 2017116447
(43) Date of publication of application: 22.04.2020
(73) Proprietor: Daio Paper Corporation, Ehime 799-0492 (JP)
(72) Inventor: YAMAMOTO, Shohei, Shikokuchuo-shi Ehime 799-0431 (JP)
(74) Representative: Meissner Bolte Partnerschaft mbB
(86) International application number: PCT/JP2018/018268
(87) International publication number: WO 2018/230215

(56) References cited:
- WO-A1-2016/029374
- JP-A- 2011 115 229
- JP-A- 2012 011 056
- JP-A- 2016 054 989
- JP-A- 2017 006 504
- US-A1- 2006 030 831
- US-A1- 2012 226 254
- US-A1- 2013 096 528

## Description

### Technical Field

The present invention relates to an underpants-type disposable diaper.

### Background Art

In general, an underpants-type disposable diaper includes an outer member forming at least lower torso portions of a front body and a back body, and an inner member attached to the outer member so as to extend from the front body to the back body and including an absorber, and has a waist opening and a pair of left and right leg openings formed by bonding both side edge portions of the outer member of the front body to both side edge portions of the outer member of the back body to form a side seal portion.

In such an underpants-type disposable diaper, in order to improve fitting to a body, the outer member has a laminated structure having a plurality of sheet layers, and various elastic members are attached in a stretched state between the sheet layers. Particularly, an underpants-type disposable diaper in which elongated elastic members along a width direction are disposed at intervals in the front-back direction in a stretched state in a width direction in a lower torso region defined as a front-back direction region corresponding to the side seal portion or in an intermediate region located between the front and back lower torso regions has relatively high fitting to a body.

As a form of an underpants-type disposable diaper, an underpants-type disposable diaper which includes an outer member cylindrically formed by bonding both sides of a rectangular front outer member to both sides of a rectangular back outer member with a side seal portion, and an inner member for absorbing excrement, disposed from the front outer member to an inner surface of the back outer member, and in which the front outer member and the back outer member are separated from each other in the front-back direction without being continuous on a crotch side, is known (see, for example, Patent Literatures 1 and 2). Such an outer member separated type diaper has an advantage that the outer member does not have to be cut off, or only a small area needs to be cut off even if the outer member is cut off in order to form a leg opening. That is, separated pieces (trims) are discarded, and therefore waste of the material (trim loss) can be suppressed advantageously. Other forms of underpants-type disposable diapers are disclosed, for example, in US 2006/030831 A1, US 2012/226254 A1, US 2013/096528 A1 and WO 2016/029374 A1. These documents further define, among other things, the formation of the buttock cover and the attachment to other components of the diapers.

In a general outer member separated type underpants-type disposable diaper, the back outer member has a longer front-back direction size than the front outer member in order to cover a lower portion of a gluteal region, and the general outer member separated type underpants-type disposable diaper has a gluteal cover portion extending closer to the center in the front-back direction than the side seal portion. It is also known that a cover portion elastic member along the width direction is disposed in the gluteal cover portion to improve fitting of the gluteal cover portion by the cover portion elastic member.

However, in the conventional outer member separated type underpants-type disposable diaper, a side edge of the gluteal cover portion and an edge on the leg opening side are unlikely to incline obliquely downward toward the center in the width direction in a wearing state, and an appearance is deteriorated disadvantageously, for example, on account of a conspicuous corner of the side edge of the gluteal cover portion on the leg opening side.

### Citation List

### Patent Literature

Patent Literature 1: JP 2008-508082 A
Patent Literature 2: JP 2012-075801 A

### Summary of Invention

### Technical Problem

Therefore, a main object of the present invention is, for example, to prevent deterioration of an appearance on account of a conspicuous corner of the side edge of the gluteal cover portion on the leg opening side in the outer member separated type underpants-type disposable diaper.

### Solution to Problem

Typical aspects for solving the above problem are as follows.

### <First aspect>

An underpants-type disposable diaper including: a rectangular front outer member forming at least a lower torso portion of a front body and a rectangular back outer member forming at least a lower torso portion of a back body independently, the front outer member and the back outer member being separated from each other at the front-back direction intermediate portion in the front-back direction,
an inner member including an absorber extending in the front-back direction from the front outer member to the back outer member, and being bonded to the front outer member and the back outer member, and
a side seal portion to which both sides of the front outer member and both sides of the back outer member are bonded to form a waist opening and a pair of left and right leg openings,
the back outer member having a gluteal cover portion extending closer to the center in the front-back direction than the side seal portion, the front-back direction length of the back outer member being 1.1 to 1.5 times the front-back direction length of the side seal portion, and the gluteal cover portion having three or more elongated cover portion elastic members along a width direction at intervals in the front-back direction at least in a region located closer to both sides in the width direction than the inner member, and the gluteal cover portion being contracted toward a center in the width direction by the cover portion elastic members,
in which a cover portion elastic member located closest to the waist opening is disposed at an end portion of the gluteal cover portion on the waist opening side, and a cover portion elastic member located closest to the leg opening is disposed at an end portion of the gluteal cover portion on the leg opening side, and
when the central interval between the cover portion elastic member located closest to the waist opening and the cover portion elastic member located second from the waist opening is represented by d_{A}, and the front-back direction length of the gluteal cover portion is represented by d_{E}, d_{A} > 0.5d_{E} is satisfied.

### (Action and effect)

A portion located closer to the waist opening than the gluteal cover portion forms a lower torso portion continuous with the front outer member through the side seal portion, and is stretched to a lateral side at the time of wearing. Therefore, an end portion of the gluteal cover portion adjacent thereto on the waist opening side is also stretched to a lateral side so as to be pulled by a portion located closer to the waist opening than the gluteal cover portion. Therefore, without the cover portion elastic member at an end portion of the gluteal cover portion on the waist opening side, a side edge of the gluteal cover portion is unlikely to incline obliquely downward from the vicinity of a lower end of the side seal portion toward the inner member (toward the center in the width direction). Meanwhile, when the cover portion elastic member located closest to the waist opening is disposed at an end portion of the gluteal cover portion on the waist opening side as in the present aspect, a contraction force toward the inner member side acts on the end portion of the gluteal cover portion on the waist opening side. As a result, a side edge of the gluteal cover portion inclines obliquely downward from the vicinity of a lower end of the side seal portion toward the inner member.

When the cover portion elastic members are disposed at almost the same central interval from the waist opening side of the gluteal cover portion to the leg opening side, most of the side edge of the gluteal cover portion excluding an end portion on the waist opening side moves almost in parallel toward the inner member in the substantially front-back direction, and a corner of the side edge of the gluteal cover portion on the leg opening side becomes conspicuous. Meanwhile, in the present aspect, when a central interval between a cover portion elastic member located closest to the waist opening and a cover portion elastic member located second from the waist opening is represented by d_{A}, and the front-back direction length of the gluteal cover portion is represented by d_{E}, d_{A} > 0.5d_{E} is satisfied. This means that there is only a cover portion elastic member located at an end portion of the gluteal cover portion on the waist opening side in a region including at least the upper half of the gluteal cover portion, and a plurality of cover portion elastic members is disposed in a region lower than the region. As described above, when the cover portion elastic members are disposed so as to be biased to the leg opening side, an end portion of the gluteal cover portion on the waist opening side is largely stretched to a lateral side at the time of wearing, but a portion on the leg opening side of the gluteal cover portion is hardly stretched. Therefore, a side edge of the gluteal cover portion inclines obliquely downward toward the inner member, and an edge of the gluteal cover portion on the leg opening side rises obliquely upward toward the side edge of the gluteal cover portion. At this time, an angle formed by the side edge of the gluteal cover portion and the edge of the gluteal cover portion on the leg opening side is an obtuse angle, and the gluteal cover portion has a shape capable of satisfactorily covering a round bulge of the gluteal region.

Furthermore, when a cover portion elastic member is not disposed at an end portion on the leg opening side of the gluteal cover portion, or when there is only one cover portion elastic member other than a cover portion elastic member located closest to the waist opening even if a cover portion elastic member is disposed at an end portion on the leg opening side of the gluteal cover portion, a corner of the side edge of the gluteal cover portion on the leg opening side is warped and conspicuous. Meanwhile, as in the present aspect, when there is only a cover portion elastic member located at an end portion of the gluteal cover portion on the waist opening side in a region including at least the upper half of the gluteal cover portion, and a plurality of cover portion elastic members is disposed including an end portion on the leg opening side in a region lower than the region, a corner of the side edge of the gluteal cover portion on the leg opening side is unlikely to be warped or slightly warped even if the corner is warped.

In the present aspect, by the overall shaping actions of the portions described above, almost the entire edge of the gluteal cover portion on the leg opening side inclines obliquely downward toward the center in the width direction in a wearing state, and the corner of the side edge of the gluteal cover portion on the leg opening side is not conspicuous to make an appearance good.

### <Second Aspect>

The underpants-type disposable diaper according to the first aspect, in which
when a central interval between adjacent cover portion elastic members located second or subsequently from the waist opening is represented by d_{B},
0.05d_{E} < d_{B} < 0.25d_{E} is satisfied.

### (Action and effect)

A side edge of the gluteal cover portion is wavy due to a contraction force of the cover portion elastic members located second or subsequently from the waist opening. Here, when an interval between the cover portion elastic members located second or subsequently from the waist opening is too wide, the side edge of the gluteal cover portion has a large and conspicuous wavy. Meanwhile, when the interval between the cover portion elastic members located second or subsequently from the waist opening is narrow as in the present aspect, even if the shape of the side edge of the gluteal cover portion is wavy, the wave has a small size, and is inconspicuous.

### <Third Aspect>

The underpants-type disposable diaper according to the first or second aspect, in which
when an interval between an edge of the gluteal cover portion on the waist opening side and the center of a cover portion elastic member located closest to the waist opening is represented by d_{c}, and an interval between an edge of the gluteal cover portion on the leg opening side and the center of the cover portion elastic member located closest to the leg opening is represented by d_{D},
d_{D} < d_{A} and d_{C} < d_{B} are satisfied.

### (Action and effect)

In consideration of the shaping action of the gluteal cover portion described above, a cover portion elastic member located closest to the waist opening is preferably not separated too much from the edge of the gluteal cover portion on the waist opening side, and a cover portion elastic member located closest to the leg opening is preferably not separated too much from the edge of the gluteal cover portion on the leg opening side. Therefore, the cover portion elastic members are preferably disposed at the intervals in the present aspect.

### <Fourth Aspect>

The underpants-type disposable diaper according to any one of the first to third aspects, in which
when a region of the gluteal cover portion located closer to both sides in the width direction than the inner member is divided equally into three portions of a first region, a second region, and a third region sequentially from an outside in the width direction toward a center, in the cover portion elastic member, that portion of the cover portion elastic member which is located in the first region and that portion of the cover portion elastic member which is located in the third region each have a higher stretch rate than that portion of the cover portion elastic member which is located in the second region.

### (Action and effect)

With such a configuration as in the present aspect, the gluteal cover portion bulges outward, and fitting to a gluteal region is improved.

### <Fifth Aspect>

The underpants-type disposable diaper according to any one of the first to fourth aspects, in which
the number of the cover portion elastic members is three or four.

### (Action and effect)

When the gluteal cover portion has the cover portion elastic member only at an end portion on the leg opening side or only at an end portion on the leg opening side and a portion close thereto except for an end portion on the waist opening side, as the side edge of the gluteal cover portion becomes closer to horizontal, an obtuse angle formed by the side edge of the gluteal cover portion and the edge of the gluteal cover portion on the leg opening side becomes larger to make the appearance of the gluteal cover portion better.

### Advantageous Effects of Invention

As described above, according to the present invention, for example, deterioration of an appearance on account of a conspicuous corner of the side edge of the gluteal cover portion on the leg opening side can be prevented advantageously in the outer member separated type underpants-type disposable diaper.

### Brief Description of Drawings

Fig. 1 is a plan view illustrating an inner surface of an underpants-type disposable diaper in an unfolded state.
Fig. 2 is a plan view illustrating an outer surface of the underpants-type disposable diaper in an unfolded state.
Fig. 3 is a cross-sectional view cut along 2-2 of Fig. 1.
Fig. 4 is a cross-sectional view cut along 3-3 of Fig. 1.
Fig. 5(a) is a cross-sectional view cut along 4-4 of Fig. 1, and Fig. 5(b) is a cross-sectional view cut along 5-5 of Fig. 1.
Fig. 6 is a perspective view of the underpants-type disposable diaper.
Fig. 7 is a plan view illustrating only a main part of an outer surface of the underpants-type disposable diaper in an unfolded state.
Fig. 8 is a plan view illustrating an outer surface of an inner member in an unfolded state together with an outline of an outer member.
Fig. 9 is a perspective view illustrating a main part of the underpants-type disposable diaper.
Fig. 10 is a perspective view illustrating a main part of the underpants-type disposable diaper.
Fig. 11 is a perspective view illustrating a main part of the underpants-type disposable diaper.

### Description of Embodiments

Hereinafter, an embodiment of the present invention will be described in detail with reference to the attached drawings. A dotted pattern portion in the cross-sectional views illustrates an adhesive as a bonding means for bonding constituent members located on a front surface side and a back surface side, and is formed by applying a hot melt adhesive by solid application, bead application, curtain application, summit application, spiral application, pattern coating (transfer of a hot melt adhesive by a letterpress method), or the like. A fixing portion of an elastic member is formed, instead of this or in addition to this, by application to an outer peripheral surface of an elastic member by a comb gun, SureWrap application, or the like. Examples of the hot melt adhesive include an EVA-based agent, a pressure-sensitive rubber-based agent (elastomer-based agent), a polyolefin-based agent, and a polyester/polyamide-based agent, and these can be used without particular limitation. As a bonding means for bonding constituent members, a means by material welding such as heat sealing or ultrasonic sealing can also be used.

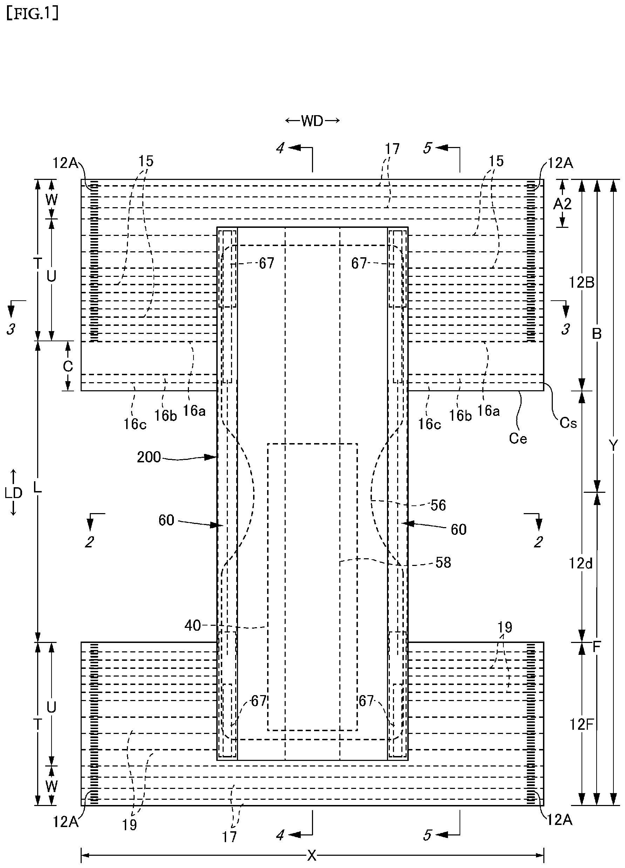

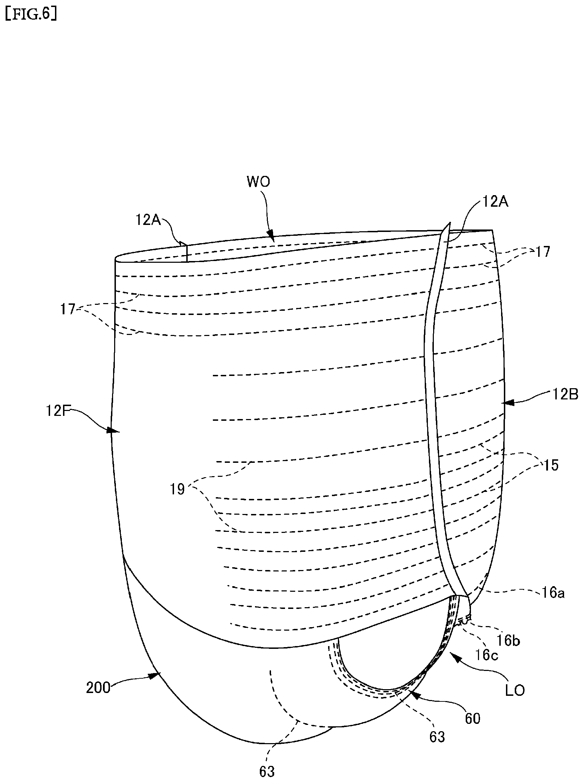

Figs. 1 to 6 illustrate an example of an underpants-type disposable diaper. The underpants-type disposable diaper includes: a rectangular front outer member 12F forming at least a lower torso portion of a front body F; a rectangular back outer member 12B forming at least a lower torso portion of a back body B; and an inner member 200 disposed inside the outer members 12F and 12B so as to extend from the front outer member 12F to the back outer member 12B through a crotch portion. Both sides of the front outer member 12F and both sides of the back outer member 12B are bonded to each other to form a side seal portion 12A. As a result, an opening formed by the front and back end portions of the outer members 12F and 12B is a waist opening WO through which a wearer's torso passes, and a portion surrounded by lower edges of the outer members 12F and 12B and a side edge of the inner member 200 on both sides of the inner member 200 in the width direction is a leg opening LO through which a leg passes. The inner member 200 is a portion for absorbing and holding excrement such as urine, and the outer members 12F and 12B are portions for supporting the inner member 200 with respect to the body of a wearer. A reference numeral Y represents the maximum length of the diaper in an unfolded state (front-back direction length from an edge of a waist opening WO of the front body F to an edge of a waist opening WO of the back body B), and a reference numeral X represents the maximum width of the diaper in an unfolded state.

The underpants-type disposable diaper in the present form has a lower torso region T defined as a front-back direction range (front-back direction range from the waist opening WO to an upper end of the leg opening LO) having the side seal portion 12A, and an intermediate region L defined as a front-back direction range of a portion forming the leg opening LO (between a front-back direction region having the side seal portion 12A of the front body F and a front-back direction region having the side seal portion 12A of the back body B). The lower torso region T can be divided into a "waist portion" W conceptually forming an edge of the waist opening and an "under-waist portion" U which is a portion lower than the waist portion W. Usually, in a case where the lower torso region T has a boundary in which a stretching stress in a width direction WD changes (for example, the fineness of an elastic member or the stretch rate thereof changes), a portion closer to the waist opening WO than the boundary closest to the waist opening WO is the waist portion W. In a case where there is no such a boundary, a waist extended portion 12E extending so as to be closer to the waist opening WO than the absorber 56 or the inner member 200 is the waist portion W. The front-back direction length varies depending on the size of a product and can be appropriately determined. For example, the length of the waist portion W can be 15 to 40 mm, and the length of the under-waist portion U can be 65 to 120 mm. Meanwhile, both side edges of the intermediate region L are each narrowed in a substantially U shape or a curved shape so as to follow a periphery of a wearer's leg, and the wearer's leg passes therethrough. As a result, the underpants-type disposable diaper in an unfolded state has an approximately hourglass shape as a whole.

### (Inner member)

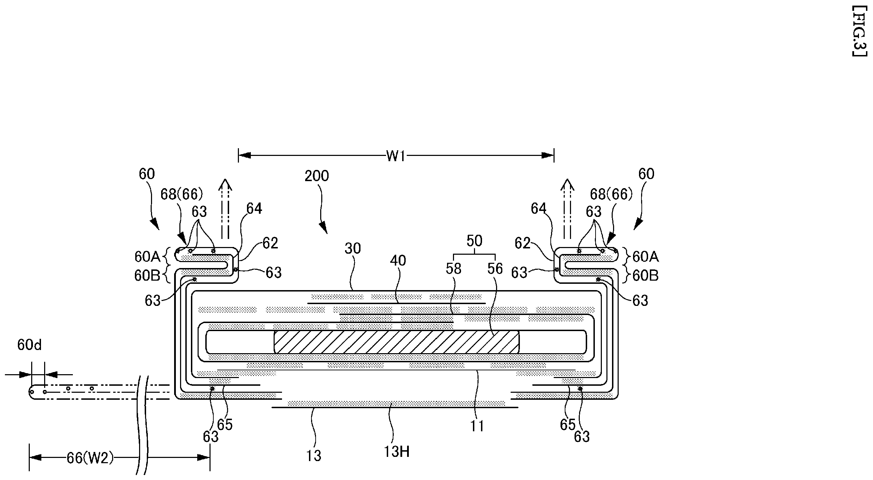

The inner member 200 can adopt an arbitrary shape, but is rectangular in the illustrated form. As illustrated in Figs. 3 to 5, the inner member 200 includes a top sheet 30 to become a body side, a liquid impervious sheet 11, and an absorbent element 50 interposed therebetween, and is a main unit portion having an absorption function. A reference numeral 40 represents an intermediate sheet (second sheet) disposed between the top sheet 30 and the absorbent element 50 in order to rapidly transfer a liquid that has passed through the top sheet 30 to the absorbent element 50. A reference numeral 60 represents a side gather 60 extending so as to come into contact with a periphery of a wearer's leg from both sides of the inner member 200 in order to prevent leakage of excrement into both sides of the inner member 200.

### (Top sheet)

The top sheet 30 transmits a liquid, and examples thereof include a perforated or imperforated nonwoven fabric and a porous plastic sheet. Among these materials, the nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include known methods such as a spunlacing method, a spunbonding method, a thermal bond method, a melt blown method, a needle punching method, an air through method, and a point bond method. For example, if softness and drapeability are demanded, a spunbonding method and a spunlacing method are preferable processing methods. If bulkiness and softness are demanded, an air through method, a point bond method, and a thermal bond method are preferable processing methods.

The top sheet 30 may be formed of a single sheet or a laminated sheet obtained by bonding two or more sheets to each other. Similarly, the top sheet 30 may be formed of a single sheet or two or more sheets in a plane direction.

Both sides of the top sheet 30 may be folded back to a back surface side at a side edge of the absorbent element 50 or may protrude from the side edge of the absorbent element 50 to a lateral side without being folded back.

For the purpose of preventing positional deviation with respect to a back surface side member or the like, it is desirable that the top sheet 30 is fixed to a member adjacent to the back surface side by a bonding means by material welding such as heat sealing or ultrasonic sealing, or with a hot melt adhesive. In the illustrated example, the top sheet 30 is fixed to a surface of an intermediate sheet 40 and a surface of a portion located on a front surface side of the absorber 56 in a wrapping sheet 58 with a hot melt adhesive applied to a back surface thereof.

### (Intermediate sheet)

In order to quickly transfer a liquid that has passed through the top sheet 30 to the absorber, it is possible to dispose the intermediate sheet (also referred to as "second sheet") 40 having a higher liquid transmission rate than the top sheet 30. The intermediate sheet 40 is used in order to rapidly transfer a liquid to the absorber to enhance absorption performance of the absorber, and to prevent a "returning" phenomenon of the absorbed liquid from the absorber. The intermediate sheet 40 can be omitted.

Examples of the intermediate sheet 40 include a similar material to that of the top sheet 30, a spunlaced nonwoven fabric, a spunbonded nonwoven fabric, an SMS nonwoven fabric, a pulp nonwoven fabric, a mixed sheet of pulp and rayon, point bonded nonwoven fabric, and crepe paper. In particular, an air through nonwoven fabric is preferable because of being bulky. As the air through nonwoven fabric, a composite fiber having a core-sheath structure is preferably used. In this case, a resin used for the core may be polypropylene (PP) but is preferably polyester (PET) having high rigidity. The basis weight is preferably 17 to 80 g/m², and more preferably 25 to 60 g/m². A raw material fiber of the nonwoven fabric preferably has a fineness of 2.0 to 10 dtex. In order to make the nonwoven fabric bulky, as mixed fibers of all or some of raw material fibers, eccentric fibers having no core in the center, hollow fibers, eccentric and hollow fibers are also preferably used.

The intermediate sheet 40 in the illustrated example is disposed at the center so as to be shorter than the width of the absorber 56, but may be disposed over the maximum width. The front-back direction length of the intermediate sheet 40 may be the same as the maximum length of the diaper, may be the same as the length of the absorbent element 50, or may be within a short length range centered on a liquid receiving region.

For the purpose of preventing positional deviation with respect to a back surface side member or the like, it is desirable that the intermediate sheet 40 is fixed to a member adjacent to the back surface side by a bonding means by material welding such as heat sealing or ultrasonic sealing, or with a hot melt adhesive. In the illustrated example, the intermediate sheet 40 is fixed to a surface of a portion located on a front surface side of the absorber 56 in the wrapping sheet 58 with a hot melt adhesive applied to a back surface thereof.

### (Liquid impervious sheet)

A material of the liquid impervious sheet 11 is not particularly limited, but examples thereof include a plastic film formed of a polyolefin-based resin such as polyethylene or polypropylene, a laminated nonwoven fabric having a plastic film disposed on a surface of a nonwoven fabric, and a laminated sheet obtained by superposing and bonding a nonwoven fabric or the like to a plastic film. For the liquid impervious sheet 11, it is preferable to use a liquid impervious and moisture pervious material favorably used from a viewpoint of preventing stuffiness. As a moisture pervious plastic film, a microporous plastic film obtained by kneading an inorganic filler in a polyolefin-based resin such as polyethylene or polypropylene, molding a sheet, and then stretching the sheet in a monoaxial or biaxial direction is widely used. In addition, a nonwoven fabric using a micro denier fiber, a nonwoven fabric that has reinforced leakproofness by reducing a space between fibers by applying heat and pressure, and a sheet that has become liquid impervious without using a plastic film by a method for applying a super absorbent polymer, a hydrophobic resin, or a water repellent agent can be used as the liquid impervious sheet 11. However, it is desirable to use a resin film in order to obtain sufficient bonding strength at the time of bonding to a cover nonwoven fabric 13 described later through a hot melt adhesive.

The liquid impervious sheet 11 may have a width housed in a back surface side of the absorbent element 50 as illustrated in the drawing, or may go around both sides of the absorbent element 50 and extend to both sides of a side surface of the top sheet 30 of the absorbent element 50 in order to enhance leakproofness. The extending portion appropriately has a width of about 5 to 20 mm on each of the left and the right.

On an inner side of the liquid impervious sheet 11, in particular, on a side surface of the absorber 56, an excretion indicator that changes a color due to absorption of a liquid can be disposed.

### (Side gather)

The side gather 60 extends along both sides of the inner member 200 over the entire front-back direction LD and is disposed in order to prevent side leakage by being in contact with the periphery of a wearer's leg, and includes what is generally called a three-dimensional gather or a plane gather.

The side gather 60 illustrated in Figs. 1, 3, and 4 is a so-called three-dimensional gather, and rises from a side of the inner member 200 to a front surface side. In the side gather 60, a root side portion 60B rises obliquely toward the center in the width direction, and a tip side portion 60A of the intermediate portion rises obliquely outward in the width direction. However, the side gather 60 is not limited thereto, and can be changed appropriately. For example, the side gather 60 can rise toward the center in the width direction as a whole.

More specifically, the side gather 60 in the illustrated example is formed by folding back a belt-shaped gather nonwoven fabric 62 having a length equal to the front-back direction length of the inner member 200 in the width direction WD at a tip portion to be folded in two, and fixing a plurality of elongated gather elastic members 63 to the folded portion and between the sheets near the folded portion in a stretched state in a longitudinal direction at intervals in the width direction WD. A base portion of the side gather 60 opposite to a tip portion thereof (an end portion opposite to the sheet-folded portion in the width direction WD) is a root portion 65 fixed to a side of a back surface side of the liquid impervious sheet 11 in the inner member 200, and a portion other than the root portion 65 is a main unit portion 66 (portion on the folded portion side) extending from the root portion 65. The main unit portion 66 has the root side portion 60B extending toward the center in the width direction, and the tip side portion 60A folded back at a tip of the root side portion 60B and extending outward in the width direction. In this form, the surface contact type side gather 60 is adopted. However, a line contact type side gather 60 not folded back outward in the width direction can also be adopted. Front-back direction both end portions of the main unit portion 66 are fallen portions 67 fixed to a side surface of the top sheet 30 in a state of falling down. Meanwhile, a front-back direction intermediate portion located therebetween is a non-fixed free portion 68. The gather elastic member 63 in the front-back direction LD is fixed in a stretched state at least to a tip portion of the free portion 68.

In the side gather 60 configured as described above, a contraction force of the gather elastic member 63 acts so as to bring the front-back direction both end portions closer to each other. However, the front-back direction both end portions of the main unit portion 66 are fixed so as not to rise, whereas a portion therebetween is the non-fixed free portion 68. Therefore, only the free portion 68 rises so as to come into contact with a body side as illustrated by the arrow in Fig. 3. In particular, when the root portion 65 is located on a back surface side of the inner member 200, the free portion 68 rises so as to open outward in the width direction at a crotch portion and in the vicinity thereof. Therefore, the side gather 60 comes into contact with a periphery of a leg with a surface to improve fitting.

Like the side gather 60 in the illustrated example, in a bent form in which the main unit portion 66 includes the root side portion 60B extending toward the center in the width direction and the tip side portion 60A folded back at a tip of the root side portion 60B and extending outward in the width direction, the tip side portion 60A is bonded to the root side portion 60B in a state of falling down at the fallen portion 67, and the root side portion 60B is bonded to the top sheet 30 in a state of falling down. For bonding facing surfaces to each other in the fallen portion 67, at least one of a hot melt adhesive by various application methods and a means by material welding such as heat sealing or ultrasonic sealing can be used. In this case, bonding of the root side portion 60B to the top sheet 30 and bonding of the tip side portion 60A to the root side portion 60B may be performed by the same means or by different means. For example, it is one preferable form to bond the root side portion 60B to the top sheet 30 with a hot melt adhesive, and to bond the tip side portion 60A to the root side portion 60B by material welding.

As the gather nonwoven fabric 62, a product obtained by subjecting a soft nonwoven fabric having excellent uniformity and concealability, such as a spunbonded nonwoven fabric (SS, SSS, or the like), an SMS nonwoven fabric (SMS, SSMMS, or the like), or a melt blown nonwoven fabric, to a water repellent treatment with silicon or the like as necessary can be used suitably. The gather nonwoven fabric 62 preferably has a fiber basis weight of about 10 to 30 g/m². As the gather elastic member 63, a rubber thread or the like can be used. When a spandex rubber thread is used, the spandex rubber thread preferably has a fineness of 470 to 1240 dtex, preferably 620 to 940 dtex. The rubber thread preferably has a stretch rate of 150 to 350%, preferably 200 to 300% at the time of fixing. Note that the term "stretch rate" means a value obtained when a natural length is assumed to be 100%. As illustrated in the drawing, a waterproof film 64 can be interposed between the two portions obtained by folding the gather nonwoven fabric 62, and in this case, the gather nonwoven fabric 62 can be partially omitted in a portion where the waterproof film 64 is present. However, in order to impart a cloth-like appearance and a cloth-like texture to a product, at least an outer surface from a base end of the side gather 60 to a tip thereof needs to be formed of the gather nonwoven fabric 62 as in the illustrated example.

The number of the gather elastic members 63 disposed in the free portion of the side gather 60 is preferably two to six, and more preferably three to five. A disposition interval 60d is suitably 3 to 10 mm. With such a configuration, a range where the gather elastic member 63 is disposed easily comes into contact with a skin with a surface. The gather elastic member 63 may be disposed not only on a tip side but also on a root side.

In the free portion 68 of the side gather 60, for bonding an inner layer and an outer layer of the gather nonwoven fabric 62 to each other or fixing the gather elastic member 63 sandwiched therebetween, at least one of a hot melt adhesive by various application methods and a fixing means by material welding such as heat sealing or ultrasonic sealing can be used. When the entire surfaces of the inner layer and the outer layer of the gather nonwoven fabric 62 are bonded to each other, softness is impaired. Therefore, preferably, a portion other than a bonded portion of the gather elastic member 63 is not bonded or weakly bonded. In the illustrated example, by applying a hot melt adhesive only to an outer peripheral surface of the gather elastic member 63 by an application means such as a comb gun or a SureWrap nozzle, and sandwiching the gather elastic member 63 between the inner layer and the outer layer of the gather nonwoven fabric 62, the gather elastic member 63 is fixed to the inner layer and the outer layer of the gather nonwoven fabric 62, and the inner layer and the outer layer of the gather nonwoven fabric 62 are fixed to each other only with the hot melt adhesive applied to the outer peripheral surface of the gather elastic member 63.

Similarly, for fixing the waterproof film 64 incorporated in the side gather 60 and the gather nonwoven fabric 62 and fixing the fallen portion 67, at least one of a hot melt adhesive by various application methods and a means by material welding such as heat sealing or ultrasonic sealing can be used.

The size of the side gather 60 in the illustrated example can be appropriately determined. However, in a case of a baby disposable diaper, for example, as illustrated in Fig. 3, a rising height of the side gather 60 (width direction length of the main unit portion 66 in an unfolded state) W2 is preferably 15 to 60 mm, and particularly preferably 20 to 40 mm. A separation distance W1 between innermost folded portions in a flatly folded state is preferably 60 to 190 mm, and particularly preferably 70 to 140 mm such that the side gather 60 is parallel to a surface of the top sheet 30.

### (Absorbent element)

The absorbent element 50 includes the absorber 56 and the wrapping sheet 58 wrapping the entire absorber 56. The wrapping sheet 58 can also be omitted.

### (Absorber)

The absorber 56 can be formed by an assembly of fibers. As this fiber assembly, in addition to those obtained by accumulating short fibers such as fluff pulps or synthetic fibers, a filament assembly obtained by opening a tow (fiber bundle) of synthetic fibers such as cellulose acetate as necessary can also be used. In a case where fluff pulps or short fibers are accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, the filament may be formed of non-crimped fibers but is preferably formed of crimped fibers. The degree of crimp of the crimped fibers may be, for example, about 5 to 75, preferably 10 to 50, and more preferably 15 to 50 per 2.54 cm. A uniformly crimped fiber is often used. In the absorber 56, super absorbent polymer particles are preferably dispersed and held.

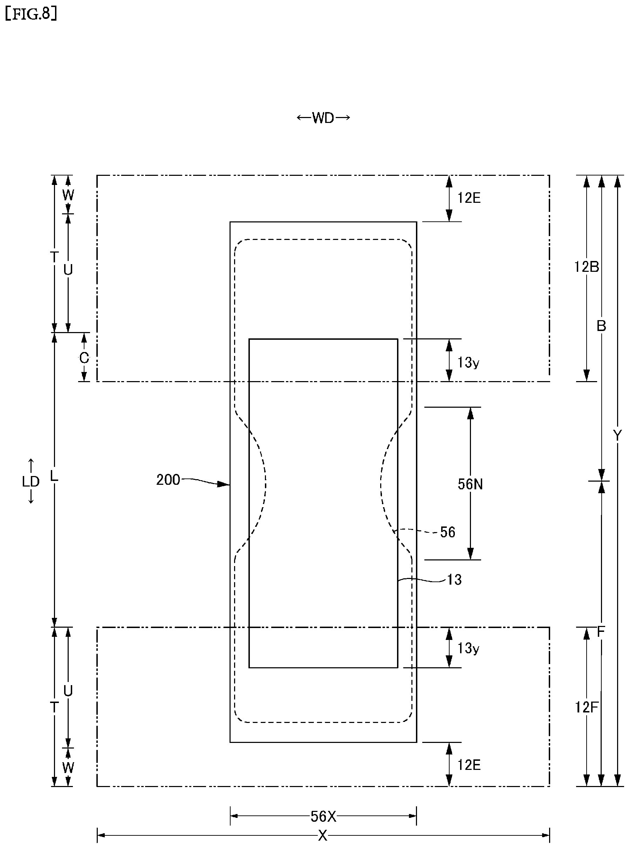

The absorber 56 may have a rectangular shape. However, as illustrated in Fig. 8 and the like, the absorber 56 preferably has an hourglass shape having a narrower portion 56N with a narrower width than front-back direction both sides thereof in a front-back direction intermediate portion because fitting of the absorber 56 itself and the side gather 60 to a periphery of a leg is improved.

The size of the absorber 56 can be determined appropriately as long as the absorber 56 extends to the front, back, left, and right of a ureteral port position. However, the absorber 56 preferably extends to peripheral edges of the inner member 200 or the vicinity thereof in the front-back direction LD and the width direction WD. Note that a reference numeral 56X represents the maximum width of the absorber 56.

### (Super absorbent polymer particles)

The absorber 56 may contain super absorbent polymer particles partially or entirely. The super absorbent polymer particles include "powder" in addition to "particles". As super absorbent polymer particles 54, those used for this type of disposable diaper can be used as they are. For example, when sieving using a standard sieve of 500 um (JIS Z8801-1: 2006) (shake for five minutes) is performed, particles in which a ratio of particles remaining on the sieve is 30% by weight or less are desirable. When sieving using a standard sieve of 180 um (JIS Z8801-1: 2006) (shake for five minutes) is performed, particles in which a ratio of particles remaining on the sieve is 60% by weight or more are desirable.

A material of the super absorbent polymer particles can be used without particular limitation, but those having a water absorption capacity of 40 g/g or more are preferable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having a water absorption rate of 70 seconds or less, particularly 40 seconds or less are suitably used. When the water absorption rate is too slow, so-called returning that a liquid supplied into the absorber 56 returns out of the absorber 56 tends to occur.

As the super absorbent polymer particles, those having a gel strength of 1000 Pa or more are suitably used. This makes it possible to effectively suppress sticky feeling after liquid absorption even in a case of using the bulky absorber 56.

The basis weight of the super absorbent polymer particles can be appropriately determined depending on the absorption amount required for an application of the absorber 56. Therefore, the basis weight can be 50 to 350 g/m² although this cannot be applied generally. The basis weight of a polymer of less than 50 g/m² makes it difficult to secure the absorption amount. The basis weight of more than 350 g/m² saturates an effect.

The spray density or the spray amount of the super absorbent polymer particles in a planar direction of the absorber 56 can be adjusted if necessary. For example, the spray amount at a liquid excretion site can be larger than that at another site. When a gender difference is considered, the spray density (amount) at a front side can be increased for men, and the spray density (amount) at a central portion can be increased for women. It is also possible to locally dispose a portion where no polymer is present (for example, in a spot shape) in a planar direction of the absorber 56.

### (Wrapping sheet)

In a case where the wrapping sheet 58 is used, as a material thereof, tissue paper, particularly, crepe paper, a nonwoven fabric, a polylaminated nonwoven fabric, a sheet with small holes, and the like can be used. However, it is desirable that the wrapping sheet 58 is a sheet from which super absorbent polymer particles do not escape. In a case where a nonwoven fabric is used instead of crepe paper, a hydrophilic SMS nonwoven fabric (SMS, SSMMS, or the like) is particularly suitable, and polypropylene, a polyethylene/polypropylene composite material, or the like can be used as a material thereof. A nonwoven fabric having a basis weight of 5 to 40 g/m², particularly of 10 to 30 g/m² is desirable.

A wrapping mode of the wrapping sheet 58 can be determined appropriately. However, a form is preferable in which the wrapping sheet 58 is wound around the absorber 56 cylindrically so as to surround front and back surfaces and both side surfaces of the absorber 56, the front and back end portions of the wrapping sheet 58 are caused to protrude from the front and back of the absorber 56, and a wound and overlapping portion and an overlapping portion of the front and back protruding portions are bonded with a hot melt adhesive or by a bonding means such as material welding from viewpoints of ease of manufacture, prevention of leakage of the super absorbent polymer particles from front and back edges, and the like.

### (Outer member)

The outer members 12F and 12B are not formed as an integral outer member passing a crotch from the front body F to the back body B, but are formed of the rectangular front outer member 12F forming at least a lower torso portion of the front body F and the rectangular back outer member 12B forming at least a lower torso portion of the back body B. The front outer member 12F and the back outer member 12B are not continuous on a crotch side, and are separated from each other in the front-back direction LD. A separation distance thereof 12d can be about 150 to 250 mm, for example.

The outer members 12F and 12B each have a lower torso portion which is a front-back direction range corresponding to the lower torso region T. In the present form, the front-back direction size of the back outer member 12B is longer than that of the front outer member 12F, and the front outer member 12F does not have a portion corresponding to the intermediate region L, but the back outer member 12B has a gluteal cover portion C extending from the lower torso region T toward the intermediate region L. Although not illustrated, also in the front outer member 12F, an inguinal cover portion extending from the lower torso region T toward the intermediate region L may be disposed.

The length of the back outer member 12B in the front-back direction LD is 1.1 to 1.5 times the length of the side seal portion 12A in the front-back direction LD. Disposition of cover portion elastic members 16a, 16b, and 16c described later is particularly effective for such a large sized underpants-type disposable diaper. Specifically, disposition of the cover portion elastic members 16a, 16b, and 16c described later is particularly effective when a length d_{E} of the gluteal cover portion C in the front-back direction LD is 10 to 100 mm, and particularly 20 to 60 mm.

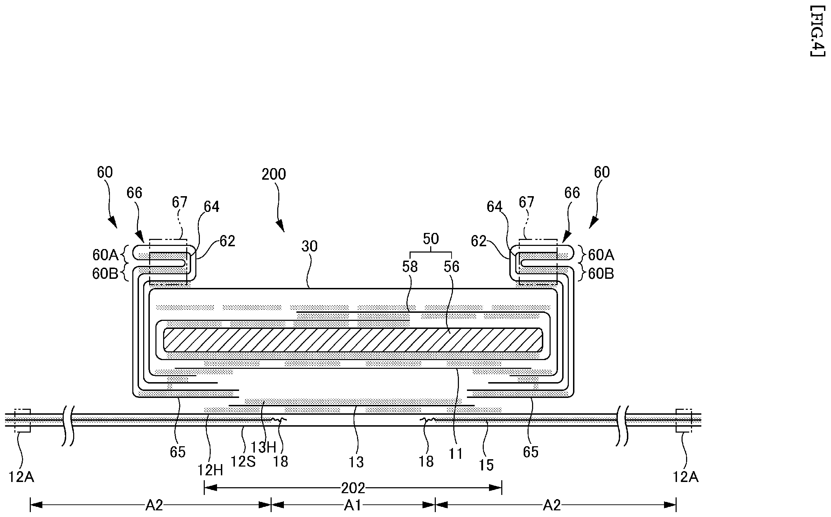

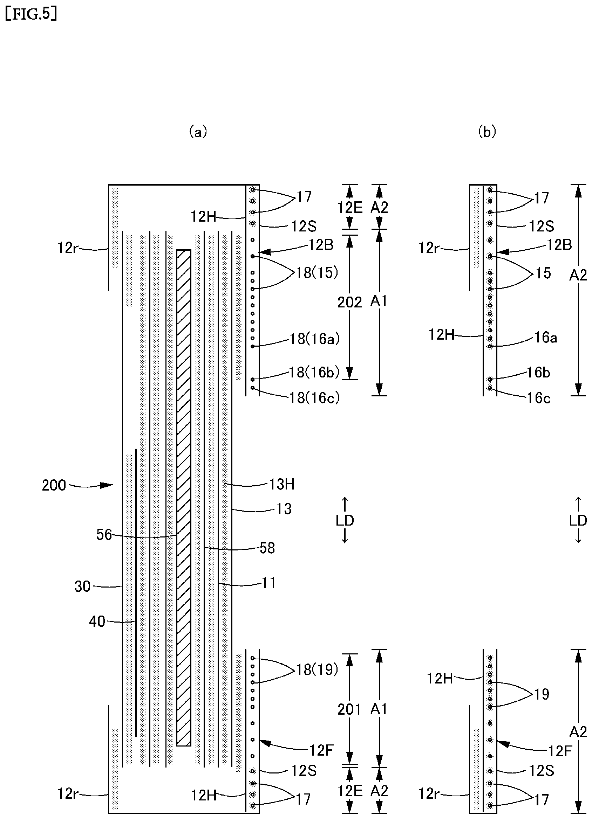

As illustrated in Figs. 4 and 5, the outer members 12F and 12B are formed by bonding an outer sheet layer 12S and an inner sheet layer 12H located on an outer side and an inner side of elastic members 15 to 19 described later, respectively, by a bonding means such as a hot melt adhesive or welding. A sheet material forming the outer sheet layer 12S and a sheet material forming the inner sheet layer 12H may be formed of a common single sheet material or may be formed of individual sheet materials. That is, in the former case, the inner sheet layer 12H and the outer sheet layer 12S are formed by an inner portion and an outer portion of a single sheet of a sheet material folded back at an edge of the waist opening WO (which may be an edge on a crotch side) in a part or the whole of the outer member, respectively. Incidentally, in the former form, the number of materials of the sheet material is small advantageously, and in the latter form, positional deviation is unlikely to occur when the inner sheet layer 12H and the outer sheet layer 12S are bonded to each other. The illustrated example corresponds to the latter, and the sheet material forming the inner sheet layer 12H extends only to an edge of the waist opening WO. However, the sheet material forming the outer sheet layer 12S goes around a waist opening WO side edge of the sheet material of the inner sheet layer 12H and is folded back inward. A folded-back portion 12r extends so as to cover an end portion of the inner member 200 on the waist opening WO side.

The sheet material used for the outer sheet layer 12S and the inner sheet layer 12H is not particularly limited, but is preferably a nonwoven fabric. Examples thereof include a nonwoven fabric formed of a synthetic fiber such as a polyolefin-based fiber including polyethylene and polypropylene, a polyester-based fiber, or a polyamide-based fiber, or a mixed fiber or a composite fiber using two or more kinds of these fibers. Furthermore, the nonwoven fabric may be manufactured by any processing. Examples of a processing method include known methods such as a spunlacing method, a spunbonding method, a thermal bond method, a melt blown method, a needle punching method, an air through method, and a point bond method. In a case where a nonwoven fabric is used, the nonwoven fabric preferably has a basis weight of about 10 to 30 g/m².

### (Stretchable region/non-stretchable region)

In each of the outer members 12F and 12B, in order to enhance fitting of a wearer to a lower torso, the elastic members 15 to 19 are disposed between the outer sheet layer 12S and the inner sheet layer 12H, and a stretchable region A2 that elastically stretches and contracts in the width direction WD along with stretching and contracting of the elastic members is formed. In the stretchable region A2, in a natural length state, the outer sheet layer 12S and the inner sheet layer 12H contract along with contraction of an elastic member to form wrinkles or pleats. When the elastic member stretches in a longitudinal direction, it is possible to stretch the outer sheet layer 12S and the inner sheet layer 12H to a predetermined stretch rate at which the outer sheet layer 12S and the inner sheet layer 12H stretch without wrinkles. As the elastic members 15 to 19, in addition to an elongated elastic member (illustrated example) such as a rubber thread, a known elastic member such as a belt-shaped member, a net-shaped member, or a film-shaped member can be used without particular limitation. As the elastic members 15 to 19, either a synthetic rubber or a natural rubber may be used.

For sticking the outer sheet layer 12S and the inner sheet layer 12H in the outer members 12F and 12B and fixing the elastic members 15 to 19 sandwiched therebetween, at least one of a hot melt adhesive by various application methods and a fixing means by material welding such as heat sealing or ultrasonic sealing can be used. When the entire surfaces of the outer members 12F and 12B are fixed rigidly, softness is impaired. Therefore, preferably, a portion other than a bonded portion of the elastic members 15 to 19 is not bonded or weakly bonded. In the illustrated example, by applying a hot melt adhesive only to outer peripheral surfaces of the elastic members 15 to 19 by an application means such as a comb gun or a SureWrap nozzle, and sandwiching the elastic members 15 to 19 between both the sheet layers 12S and 12H, the elastic members 15 to 19 are fixed to both the sheet layers 12S and 12H only with the hot melt adhesive applied to the outer peripheral surfaces of the elastic members 15 to 19, and both the sheet layers 12S and 12H are fixed to each other. The elastic members 15 to 19 can be fixed to the outer sheet layer 12S and the inner sheet layer 12H only at both end portions in a stretchable direction in a stretchable region.

The elastic members 15 to 19 in the illustrated example will be described in more detail. Between the outer sheet layer 12S and the inner sheet layer 12H in the waist portion W of the outer members 12F and 12B, a plurality of waist portion elastic members 17 is attached at intervals in a front-back direction so as to be continuous over the entire width direction WD. One or more waist portion elastic members 17 disposed in a region adjacent to the under-waist portion U may overlap with the inner member 200, or may be disposed on both sides thereof in the width direction except for the center in the width direction overlapping with the inner member 200. As the waist portion elastic member 17, it is preferable to dispose 3 to 22 rubber threads each having a fineness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber) at intervals of 4 to 12 mm. A resultant stretch rate of the waist portion W in the width direction WD is preferably 150 to 400%, and particularly preferably about 220 to 320%. In the waist portion W, all of the waist portion elastic members 17 in the front-back direction LD do not have to have the same fineness or the same stretch rate. For example, the fineness and the stretch rate of the elastic member 17 may be different between an upper portion and a lower portion of the waist portion W.

Between the outer sheet layer 12S and the inner sheet layer 12H in the under-waist portion U of the outer members 12F and 12B, a plurality of under-waist portion elastic members 15 and 19 formed of an elongated elastic member is disposed at intervals in a front-back direction.

As the under-waist portion elastic members 15 and 19, it is preferable to dispose 5 to 30 rubber threads each having a fineness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber) at intervals of 1 to 15 mm, particularly 3 to 8 mm. A resultant stretch rate of the under-waist portion U in the width direction WD is preferably 200 to 350%, and particularly preferably about 240 to 300%.

Between the outer sheet layer 12S and the inner sheet layer 12H in the gluteal cover portion C of the back outer member 12B, the cover portion elastic members 16a, 16b, and 16c formed of an elongated elastic member are attached. The gluteal cover portion C is contracted toward the center in the width direction WD by the cover portion elastic members 16a, 16b, and 16c.

As the cover portion elastic members 16a, 16b, and 16c, it is preferable to dispose a rubber thread having a fineness of 155 to 1880 dtex, particularly about 470 to 1240 dtex (in a case of a synthetic rubber, having a cross section of 0.05 to 1.5 mm², particularly about 0.1 to 1.0 mm² in a case of a natural rubber). A resultant stretch rate of the gluteal cover portion C in the width direction WD is preferably 150 to 400%, and particularly preferably about 250 to 350%.

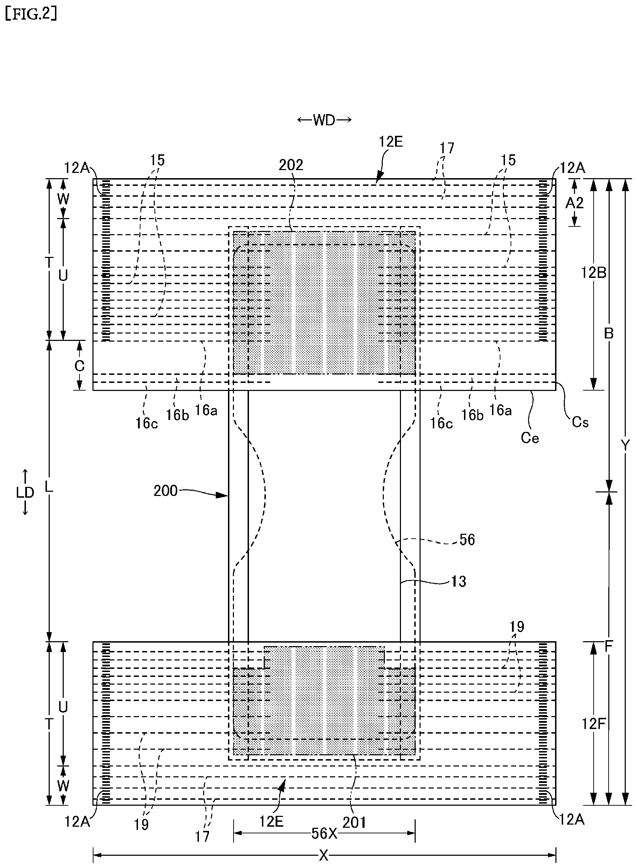

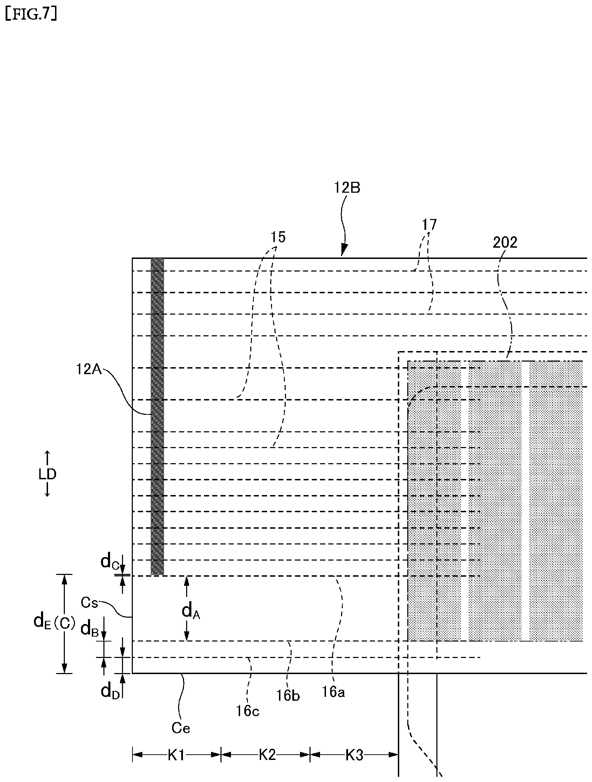

Characteristically, as illustrated in Figs. 2 and 7, in the gluteal cover portion C, the three cover portion elastic members 16a, 16b, and 16c are disposed at intervals in the front-back direction LD. The cover portion elastic member 16a located closest to the waist opening WO is disposed at an end portion of the gluteal cover portion C on the waist opening WO side, and the cover portion elastic member 16c located closest to the leg opening LO is disposed at an end portion of the gluteal cover portion C on the leg opening LO side. When a central interval between the cover portion elastic member 16a located closest to the waist opening WO and the cover portion elastic member 16b located second from the waist opening WO is represented by d_{A}, and the length of the gluteal cover portion C in the front-back direction LD is represented by d_{E}, d_{A} > 0.5d_{E} is satisfied. The number of the cover portion elastic members 16a, 16b, 16c can be appropriately determined, and is preferably 3 to 10, more preferably 3 to 5, and particularly preferably 3 or 4.

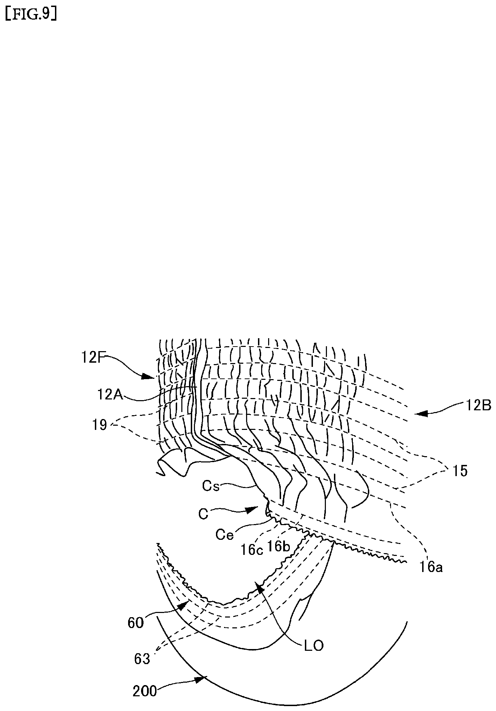

A portion located closer to the waist opening than the gluteal cover portion C forms a lower torso portion continuous with the front outer member 12F through the side seal portion 12A, and is stretched to a lateral side at the time of wearing. Therefore, an end portion of the gluteal cover portion C adjacent thereto on the waist opening WO side is also stretched to a lateral side so as to be pulled by a portion located closer to the waist opening than the gluteal cover portion C. Therefore, without the cover portion elastic members 16a, 16b, and 16c at an end portion of the gluteal cover portion C on the waist opening WO side, a side edge Cs of the gluteal cover portion C is unlikely to incline obliquely downward from the vicinity of a lower end of the side seal portion 12A toward the inner member 200 (center in the width direction WD). Meanwhile, as described above, when the cover portion elastic members 16a, 16b, and 16c located closest to the waist opening WO are disposed at an end portion of the gluteal cover portion C on the waist opening WO side, as illustrated in Fig. 9, a contraction force toward the inner member 200 acts on the end portion of the gluteal cover portion C on the waist opening WO side. As a result, the side edge Cs of the gluteal cover portion C inclines obliquely downward from the vicinity of a lower end of the side seal portion 12A toward the inner member 200.

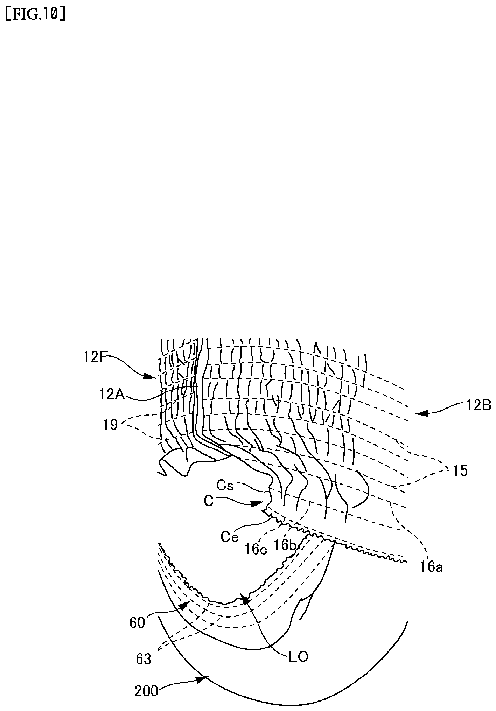

As illustrated in Fig. 10, when the cover portion elastic members 16a, 16b, and 16c are disposed at almost the same central interval from the waist opening WO side of the gluteal cover portion C to the leg opening LO side, most of the side edge Cs of the gluteal cover portion C excluding an end portion on the waist opening WO side moves almost in parallel toward the inner member 200 in the substantially front-back direction LD, and a corner of the side edge Cs of the gluteal cover portion C on the leg opening LO side becomes conspicuous. Meanwhile, in the example illustrated in Figs. 2 and 7, when a central interval between the cover portion elastic member 16a located closest to the waist opening WO and the cover portion elastic member 16b located second from the waist opening WO is represented by d_{A}, and the length of the gluteal cover portion C in the front-back direction LD is represented by d_{E}, d_{A} > 0.5d_{E} is satisfied. This means that there is only the cover portion elastic member 16a located at an end portion of the gluteal cover portion C on the waist opening WO side in a region including at least the upper half of the gluteal cover portion C, and a plurality of cover portion elastic members 16b and 16c is disposed in a region lower than the region. As described above, when the cover portion elastic members 16a, 16b, and 16c are disposed so as to be biased to the leg opening LO side, as illustrated in Fig. 9, an end portion of the gluteal cover portion C on the waist opening WO side is largely stretched to a lateral side at the time of wearing, but a portion of the gluteal cover portion C on the leg opening LO side is hardly stretched. Therefore, a side edge Cs of the gluteal cover portion C inclines obliquely downward toward the inner member 200, and an edge Ce of the gluteal cover portion C on the leg opening LO side rises obliquely upward toward the side edge of the gluteal cover portion C. At this time, an angle formed by the side edge Cs of the gluteal cover portion C and the edge Ce of the gluteal cover portion C on the leg opening LO side is an obtuse angle, and the gluteal cover portion C has a shape capable of satisfactorily covering a round bulge of the gluteal region.

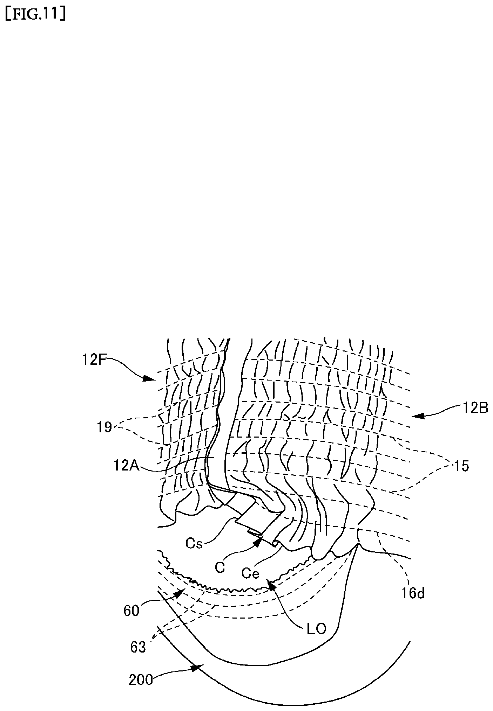

Furthermore, as illustrated in Fig. 11, even if a cover portion elastic member 16d located closest to the leg opening LO is disposed above an end portion of the gluteal cover portion C on the leg opening LO side, or even if a cover portion elastic member is disposed at an end portion of the gluteal cover portion C on the leg opening LO side (not illustrated), when there is only one cover portion elastic member other than the cover portion elastic member located closest to the waist opening WO (that is, a form in which the second cover portion elastic member 16b in the example of Figs. 2 and 7 is not disposed), a corner of the side edge Cs of the gluteal cover portion C on the leg opening LO side is warped and conspicuous. Meanwhile, as illustrated in Figs. 2 and 7, when there is only the cover portion elastic member 16a located at an end portion of the gluteal cover portion C on the waist opening WO side in a region including at least the upper half of the gluteal cover portion C, and the plurality of cover portion elastic members 16b and 16c is disposed including an end portion on the leg opening LO side in a region lower than the region, as illustrated in Fig. 9, a corner of the side edge Cs of the gluteal cover portion C on the leg opening LO side is unlikely to be warped or slightly warped even if the corner is warped.

In the form illustrated in Figs. 2 and 7, by the overall shaping actions of the portions described above, as illustrated in Fig. 9, almost the entire edge Ce of the gluteal cover portion C on the leg opening LO side inclines obliquely downward toward the center in the width direction WD in a wearing state, and the corner of the side edge Cs of the gluteal cover portion C on the leg opening LO side is not conspicuous to make an appearance good. In particular, in consideration of the shaping actions of the portions described above, when the gluteal cover portion has the cover portion elastic member only at an end portion on the leg opening side or only at an end portion on the leg opening side and a portion close thereto except for an end portion on the waist opening side, as the side edge of the gluteal cover portion becomes closer to horizontal, an obtuse angle formed by the side edge of the gluteal cover portion and the edge of the gluteal cover portion on the leg opening side becomes larger to make the appearance of the gluteal cover portion better. Therefore, more preferably, d_{A} > 0.5d_{E} is satisfied, and the number of the cover portion elastic members is three or four.

The side edge Cs of the gluteal cover portion C is wavy due to a contraction force of the cover portion elastic members 16b and 16c located second or subsequently from the waist opening WO. Here, when an interval between the cover portion elastic members 16b and 16c located second or subsequently from the waist opening WO is too wide, as illustrated in Fig. 10, the side edge Cs of the gluteal cover portion C has a large and conspicuous wavy shape.

Meanwhile, as illustrated in Figs. 2 and 7, when a central interval between the cover portion elastic members 16b and 16c located second or subsequently from the waist opening WO is represented by dB, if 0.05d_{E} < d_{B} < 0.25d_{E} is satisfied, as illustrated in Fig. 9, even if the side edge Cs of the gluteal cover portion C is wavy, the wave has a small size and is inconspicuous.

In consideration of the shaping action of the gluteal cover portion C described above, the cover portion elastic member 16a located closest to the waist opening WO is preferably not separated too much from the edge of the gluteal cover portion C on the waist opening WO side, and the cover portion elastic member 16c located closest to the leg opening LO is preferably not separated too much from the edge Ce of the gluteal cover portion C on the leg opening LO side. Therefore, when an interval between an edge of the gluteal cover portion C on the waist opening WO side and the center of the cover portion elastic member 16a located closest to the waist opening WO is represented by d_{C}, and an interval between the edge Ce of the gluteal cover portion C on the leg opening LO side and the center of the cover portion elastic member 16c located closest to the leg opening LO is represented by d_{D}, d_{D} < d_{A} and d_{C} < d_{B} are preferably satisfied. In particular, a relationship of d_{C} < d_{B} < d_{D} < d_{A} is more preferably satisfied.

When a region of the gluteal cover portion C located closer to both sides in the width direction WD than the inner member 200 is divided equally into three portions of a first region K1, a second region K2, and a third region K3 sequentially from an outside in the width direction WD toward the center, it is one preferable form that, in the cover portion elastic members 16a, 16b, and 16c, a portion located in the first region K1 and a portion located in the third region K3 each have a higher stretch rate than a portion located in the second region K2. The stretch rates of the portions located in the respective regions of the cover portion elastic members 16a, 16b, and 16c can be appropriately determined. However, the stretch rate of each of the portions located in the first region K1 and the third region K3 is preferably about 1.1 to 2 times the stretch rate of the portion located in the second region K2. As described above, by the change of the stretch rates of the cover portion elastic members 16a, 16b, and 16c in the width direction WD, the gluteal cover portion C bulges outward, and fitting to a gluteal region is improved.

Meanwhile, in a case where an inguinal cover portion is disposed in the front outer member 12F, a cover portion elastic member can be disposed similarly to the gluteal cover portion C.

In a case where the elastic members 15, 16, and 19 are disposed in a front-back direction range having the absorber 56 like the under-waist portion U and the gluteal cover portion C in the illustrated example, in order to prevent contraction of the absorber 56 in the width direction WD in a part or the whole thereof, a width direction intermediate portion including a part or the whole of a portion overlapping with the absorber 56 in the width direction WD (preferably including the whole of the inner and outer bonded portions 201 and 202) is a non-stretchable region A1, and both sides thereof in the width direction are stretchable regions A2. The waist portion W is preferably the stretchable region A2 over the entire width direction WD. However, like the under-waist portion U, the waist portion W may have the non-stretchable region A1 in a width direction intermediate portion.

The stretchable region A2 and the non-stretchable region A1 can be formed by supplying elastic members 15 to 17 and 19 between the inner sheet layer 12H and the outer sheet layer 12S, fixing the elastic members 15, 16, and 19 in at least both end portions in a stretchable direction in the stretchable region A2 through a hot melt adhesive without fixing the elastic members 15, 16, and 19 in a region to be the non-stretchable region A1, and cutting the elastic members 15, 16, and 19 at one place in a width direction intermediate portion or cutting the elastic members 15, 16, and 19 finely at many places in the region to be the non-stretchable region A1 by pressing and heating the elastic members 15, 16, and 19 to leave elasticity in the stretchable region A2 and destroying elasticity in the non-stretchable region A1. In the former case, as illustrated in Fig. 4, in the non-stretchable region A1, a cutting residue continuous from the elastic members 15, 16, and 19 in the stretchable region A2 remains between the outer sheet layer 12S and the inner sheet layer 12H while being contracted to a natural length alone as an unnecessary elastic member 18. In the latter case, although not illustrated, a cutting residue continuous from the elastic members 15, 16, and 19 in the stretchable region A2 and a cut piece of an elastic member not continuous from the elastic members 15, 16, and 19 in either of the stretchable regions A2 remain between the outer sheet layer 12S and the inner sheet layer 12H while being contracted to a natural length alone as an unnecessary elastic member.

### (Cover nonwoven fabric)

In an outer member separated type underpants-type disposable diaper, the inner member 200 is exposed between the front outer member 12F and the back outer member 12B. Therefore, in order to prevent the liquid impervious sheet 11 from being exposed to a back surface of the inner member 200, the outer member separated type underpants-type disposable diaper preferably includes a cover nonwoven fabric 13 covering the back surface of the inner member 200 from a portion between the front outer member 12F and the inner member 200 to a portion between the back outer member 12B and the inner member 200.

As a nonwoven fabric used for the cover nonwoven fabric 13, for example, a material similar to those of the outer members 12F and 12B can be appropriately selected, and the nonwoven fabric used for the cover nonwoven fabric 13 is not particularly limited by the type of a fiber or a method for bonding (interlacing) fibers. However, it is desirable to use an air through nonwoven fabric. In this case, the basis weight is preferably 20 to 40 g/m² and the thickness is preferably 0.3 to 1.0 mm. As the cover nonwoven fabric 13, an imperforated nonwoven fabric having no hole passing therethrough from the front to the back or a perforated nonwoven fabric having many holes passing therethrough from the front to the back at intervals may be used.

The front-back direction range of the cover nonwoven fabric 13 is not particularly limited, and as illustrated in Figs. 2 and 5, may extend in the front-back direction LD over the entire region from a front end to a back end of the inner member 200. As illustrated in Fig. 8, the front-back direction range of the cover nonwoven fabric 13 may extend in the front-back direction LD from a front-back direction intermediate position of a region where the front outer member 12F and the inner member 200 overlap with each other to a front-back direction intermediate position of a region where the back outer member 12B and the inner member 200 overlap with each other. In the case of the example illustrated in Fig. 8, a front-back direction length 13y of an overlapping portion between the cover nonwoven fabric 13 and the front outer member 12F and a front-back direction length 13y of an overlapping portion between the cover nonwoven fabric 13 and the back outer member 12B can be appropriately determined, but can be each about 20 to 40 mm in a usual case.

The width direction range of the cover nonwoven fabric 13 is a range in which a back surface exposed portion of the liquid impervious sheet 11 can be concealed. For this reason, in the illustrated example, the liquid impervious sheet 11 is exposed between base ends of the left and right side gathers 60. Therefore, the cover nonwoven fabric 13 is disposed so as to cover a width direction range from a back surface side of a base portion of at least one of the side gathers 60 to a back surface side of a base portion of the other of the side gathers 60. This makes it possible to conceal the liquid impervious sheet 11 with the cover nonwoven fabric 13 and the gather nonwoven fabric 62 of the side gather 60. In addition, not by covering a back surface side of the base portion of the side gather 60 with width direction both end portions of the cover nonwoven fabric 13 but by covering a back surface side of the width direction both end portions of the cover nonwoven fabric 13 with the gather nonwoven fabric 62, it is possible to conceal the liquid impervious sheet 11 with the cover nonwoven fabric 13 and the gather nonwoven fabric 62. In this case, since both sides of the cover nonwoven fabric 13 are covered with the gather nonwoven fabric 62, both sides of the cover nonwoven fabric 13 are less likely to be peeled off from the liquid impervious sheet 11 advantageously.

The inner surface and the outer surface of the cover nonwoven fabric 13 can be bonded to facing surfaces thereof via a hot melt adhesive. A fixing region of the cover nonwoven fabric 13 can extend through the entire front-back direction and the entire width direction of the cover nonwoven fabric 13 can be fixed, or a part thereof can be non-fixed. For example, when width direction both end portions of the cover nonwoven fabric 13 are non-fixed, even if a side of the absorber 56 is contracted somewhat due to an influence of the side gather 60, the influence is small, and wrinkles or creases are less likely to be formed in the cover nonwoven fabric 13 advantageously. In this case, the widths of the non-fixed portions at width direction both end portions of the cover nonwoven fabric 13 may be determined appropriately, but can be, for example, 3 to 10 mm, preferably 5 to 8 mm.

### (Inner and outer bonded portion)

The inner member 200 can be fixed to the outer members 12F and 12B by a bonding means by material welding such as heat sealing or ultrasonic sealing, or with a hot melt adhesive. In the illustrated example, via a hot melt adhesive applied to the back surface of the inner member 200, that is, the back surface of the liquid impervious sheet 11 and the root portion 65 of the side gather 60 in this case, the inner member 200 is fixed to the inner surfaces of the outer members 12F and 12B. The inner and outer bonded portions 201 and 202 for fixing the inner member 200 to the outer members 12F and 12B can be disposed in almost the entire region where the inner member 200 overlaps with the outer members 12F and 12B as illustrated in Fig. 2, and can be disposed, for example, in a portion excluding width direction both end portions of the inner member 200.

### <Explanation of terms in specification>

The following terms in the specification have the following meanings unless otherwise specified in the specification.
·"Front-back (longitudinal) direction" means a direction connecting a ventral side (front side) and a dorsal side (back side), and "width direction" means a direction orthogonal to the front-back direction (left-right direction).
·"Front surface side" means a side closer to a wearer's skin when an underpants-type disposable diaper is worn. "Back surface side" means a side far from a wearer's skin when an underpants-type disposable diaper is worn.
·"Front surface" means a surface of a member closer to a wearer's skin when an underpants-type disposable diaper is worn. "Back surface" means a surface far from a wearer's skin when an underpants-type disposable diaper is worn.
·"Stretch rate" means a value obtained when a natural length is assumed to be 100%.
·"Gel strength" is measured as follows. To 49.0 g of artificial urine (mixture of 2% by weight of urea, 0.8% by weight of sodium chloride, 0.03% by weight of calcium chloride dihydrate, 0.08% by weight of magnesium sulfate heptahydrate, and 97.09% by weight of deionized water), 1.0 g of a super absorbent polymer is added, and the resulting mixture is stirred with a stirrer. The gel thus generated is left in a thermohygrostat at 40°C × 60%RH for three hours. Thereafter, the temperature is returned to room temperature, and gel strength is measured with a curdmeter (Curdmeter-MAX ME-500 manufactured by I. Techno Engineering Co., Ltd.).
·"Basis weight" is measured as follows. A sample or a test piece is predried and then left in a test chamber or an apparatus in a standard state (test location is at a temperature of 23 ± 1°C and a relative humidity of 50 ± 2%) so as to have a constant weight. Predrying refers to causing a sample or a test piece to have a constant weight in an environment of a temperature of 100°C. Incidentally, fibers having an official moisture regain of 0.0% do not have to be predried. A sample of 100 mm × 100 mm in size is cut out from a test piece having a constant weight using a template for sampling (100 mm × 100 mm). The weight of the sample is measured. The weight is multiplied by 100 to calculate the weight per square meter to be used as a basis weight.
·"Thickness" is automatically measured under conditions that a load is 0.098 N/cm² and a pressing area is 2 cm² using an automatic thickness meter (KES-G5 handy compression measuring program).
·Water absorption capacity is measured in accordance with JIS K7223-1996 "Test method for water absorption capacity of super absorbent polymer".
·Water absorption rate is "time to end point" when JIS K7224-1996 "Test method for water absorption rate of super absorbent polymer" is performed using 2 g of super absorbent polymer and 50 g of physiological saline.
·"Unfolded state" means a flatly unfolded state without contraction or slackness.
·The size of each portion means a size not in a natural length state but in an unfolded state unless otherwise specified.
·In a case where environmental conditions in a test and a measurement are not described, the test and the measurement are performed in a test room or an apparatus in a standard state (test location is at a temperature of 23 ± 1°C and a relative humidity of 50 ± 2%).

### Industrial Applicability

The present invention is applicable to an underpants-type disposable diaper.

### Reference Signs List

- 11: Liquid impervious sheet
- 12A: Side seal portion
- 12B: Back outer member
- 12E: Waist extended portion
- 12F: Front outer member
- 12H: Inner sheet layer
- 12S: Outer sheet layer
- 13: Cover nonwoven fabric
- 16a, 16b, 16c: Cover portion elastic member
- 17: Waist portion elastic member
- 18: Unnecessary elastic member
- 200: Inner member
- 201, 202: Inner and outer bonded portion
- 30: Top sheet
- 40: Intermediate sheet
- 50: Absorbent element
- 56: Absorber
- 58: Wrapping sheet
- 60: Side gather
- 60A: Tip side portion
- 60B: Root side portion
- 62: Gather nonwoven fabric
- 67: Fallen portion
- 68: Free portion
- A1: Non-stretchable region
- A2: Stretchable region
- C: Gluteal cover portion
- L: Intermediate region
- LD: Front-back direction
- T: Lower torso region
- U: Under-waist portion
- W: Waist portion
- WD: Width direction
- WO: Waist opening
- LO: Leg opening
- Cs: Side edge
- Ce: Edge
- K1: First region
- K2: Second region
- K3: Third region

## Claims

1. An underpants-type disposable diaper comprising:
a rectangular front outer member (12F) forming at least a lower torso portion of a front body and a rectangular back outer member (12B) forming at least a lower torso portion of a back body independently, the front outer member (12F) and the back outer member (12B) being separated from each other at a front-back direction (LD) intermediate portion in the front-back direction (LD);
an inner member (200) including an absorber (56) extending in the front-back direction (LD) from the front outer member (12F) to the back outer member (12B), and being bonded to the front outer member (12F) and the back outer member (12B) respectively; and
a side seal portion (12A) to which both sides of the front outer member (12F) and both sides of the back outer member (12B) are bonded to form a waist opening (WO) and a pair of left and right leg openings (LO),
the back outer member (12B) having a gluteal cover portion (C) extending closer to a center in the front-back direction (LD) than the side seal portion (12A), a front-back direction (LD) length of the back outer member (12B) being 1.1 to 1.5 times a front-back direction (LD) length of the side seal portion (12A), and the gluteal cover portion (C) having three or more elongated cover portion elastic members (16a, 16b, 16c) along a width direction (WD) at intervals in the front-back direction (LD) at least in a region located closer to both sides in the width direction (WD) than the inner member (200), and the gluteal cover portion (C) being contracted toward a center in the width direction (WD) by the cover portion elastic members (16a, 16b, 16c),
wherein the cover portion elastic member (16a) located closest to the waist opening (WO) is disposed at an end portion of the gluteal cover portion (C) on the waist opening (WO) side, and the cover portion elastic member (16c) located closest to the leg opening (LO) is disposed at an end portion of the gluteal cover portion (C) on the leg opening (LO) side, and
when a central interval between the cover portion elastic member (16a) located closest to the waist opening (WO) and the cover portion elastic member (16b) located second from the waist opening (WO) is represented by d_{A}, and a front-back direction (LD) length of the gluteal cover portion (C) is represented by d_{E}, d_{A} > 0.5d_{E} is satisfied.

2. The underpants-type disposable diaper according to claim 1, wherein
when a central interval between adjacent cover portion elastic members (16b, 16c) located second or subsequently from the waist opening (WO) is represented by d_{B},
0.05d_{E} < d_{B} < 0.25d_{E} is satisfied.

3. The underpants-type disposable diaper according to claim 1 or 2, wherein
when an interval between an edge of the gluteal cover portion (C) on the waist opening (WO) side and a center of a cover portion elastic member (16a) located closest to the waist opening (WO) is represented by dc, and an interval between an edge (Ce) of the gluteal cover portion (C) on the leg opening (LO) side and a center of the cover portion elastic member (16c) located closest to the leg opening (LO) is represented by d_{D},
d_{D} < d_{A} and de < d_{B} are satisfied.

4. The underpants-type disposable diaper according to any one of claims 1 to 3, wherein
when a region of the gluteal cover portion (C) located closer to both sides in the width direction (WD) than the inner member (200) is divided equally into three portions of a first region (K1), a second region (K2), and a third region (K3) sequentially from an outside in the width direction (WD) toward a center, in the cover portion elastic member (16a, 16b, 16c), that portion of the cover portion elastic member (16a, 16b, 16c) which is located in the first region (K1) and that portion of the cover portion elastic member (16a, 16b, 16c) which is located in the third region (K3) each have a higher stretch rate than that portion of the cover portion elastic member (16a, 16b, 16c) which is located in the second region.

5. The underpants-type disposable diaper according to any one of claims 1 to 4, wherein
the number of the cover portion elastic members (16a, 16b, 16c) is three or four.

## Patentansprüche

1. Wegwerfwindel des Unterhosentyps, die Folgendes umfasst:
ein rechteckiges vorderes äußeres Element (12F), das wenigstens einen unteren Torsoabschnitt eines vorderen Körpers bildet, und ein rechteckiges hinteres äußeres Element (12B), das unabhängig wenigstens einen unteren Torsoabschnitt eines hinteren Körpers bildet, wobei das vordere äußere Element (12F) und das hintere äußere Element (12B) in der Richtung (LD) von vorn nach hinten bei einem dazwischenliegenden Abschnitt in einer Richtung (LD) von vorn nach hinten voneinander getrennt sind;
ein inneres Element (200), das ein Absorptionselement (56) umfasst, das in der Richtung (LD) von vorn nach hinten vom vorderen äußeren Element (12F) zum hinteren äußeren Element (12B) verläuft, und jeweils mit dem vorderen äußeren Element (12F) und dem hinteren äußeren Element (12B) verbunden ist; und
einen seitlichen Dichtungsabschnitt (12A), mit dem beide Seiten des vorderen äußeren Elements (12F) und beide Seiten des hinteren äußeren Elements (12B) verbunden sind, um eine Hüftöffnung (WO) und ein Paar linker und rechter Beinöffnungen (LO) zu bilden,
wobei das hintere äußere Element (12B) einen Gesäßbedeckungsabschnitt (C) hat, der sich in der Richtung (LD) von vorn nach hinten weiter zur Mitte als der seitliche Dichtungsabschnitt (12A) erstreckt, wobei eine Länge des hinteren äußeren Elements (12B) in einer Richtung (LD) von vorn nach hinten die 1,1-fache bis 1,5-fache Länge des seitlichen Dichtungsabschnitts (12A) in einer Richtung (LD) von vorn nach hinten ist, und wobei der Gesäßbedeckungsabschnitt (C) drei oder mehr in Längsrichtung verlaufende elastische Elemente (16a, 16b, 16c) des Bedeckungsabschnitts längs einer Breitenrichtung (WD) in Abständen in der Richtung (LD) von vorn nach hinten wenigstens in einem Bereich aufweist, der in der Breitenrichtung (WD) näher an beiden Seiten als das innere Element (200) angeordnet ist, und wobei der Gesäßbedeckungsabschnitt (C) durch die elastischen Elemente (16a, 16b, 16c) des Bedeckungsabschnitts zur Mitte in der Breitenrichtung (WD) kontrahiert wird,
wobei das elastische Element (16a) des Bedeckungsabschnitts, das sich direkt an der Hüftöffnung (WO) befindet, an einem Endabschnitt des Gesäßbedeckungsabschnitts (C) auf der Seite der Hüftöffnung (WO) angeordnet ist, und wobei das elastische Element (16c) des Bedeckungsabschnitts, das sich direkt an der Beinöffnung (LO) befindet, an einem Endabschnitt des Gesäßbedeckungsabschnitts (C) auf der Seite der Beinöffnung (LO) angeordnet ist, und
dann, wenn ein zentraler Abstand zwischen dem elastischen Element (16a) des Bedeckungsabschnitts, das sich direkt an der Hüftöffnung (WO) befindet, und dem elastischen Element (16b) des Bedeckungsabschnitts, das sich an zweiter Stelle von der Hüftöffnung (WO) aus befindet, durch d_{A} dargestellt wird, und eine Länge des Gesäßbedeckungsabschnitts (C) in einer Richtung (LD) von vorn nach hinten durch d_{E} dargestellt wird, d_{A} > 0,5d_{E} erfüllt ist.

2. Wegwerfwindel des Unterhosentyps nach Anspruch 1, wobei
dann, wenn ein zentraler Abstand zwischen aneinander angrenzenden elastischen Elementen (16b, 16c) des Bedeckungsabschnitts, die sich von der Hüftöffnung (WO) aus an zweiter Stelle oder dahinter befinden, durch d_{B} dargestellt wird, 0,05d_{E} < d_{B} < 0,25d_{E} erfüllt ist.

3. Wegwerfwindel des Unterhosentyps nach Anspruch 1 oder 2, wobei
dann, wenn ein Abstand zwischen einer Kante des Gesäßbedeckungsabschnitts (C) auf der Seite der Hüftöffnung (WO) und einer Mitte eines elastischen Elements (16a) des Bedeckungsabschnitts, das sich direkt an der Hüftöffnung (WO) befindet, durch dc dargestellt wird, und ein Abstand zwischen einer Kante (Ce) des Gesäßbedeckungsabschnitts (C) auf der Seite der Beinöffnung (LO) und der Mitte des elastischen Elements (16c) des Bedeckungsabschnitts, das sich direkt an der Beinöffnung (LO) befindet, durch d_{D} dargestellt wird, d_{D} < d_{A} und dc < d_{B} erfüllt sind.

4. Wegwerfwindel des Unterhosentyps nach einem der Ansprüche 1 bis 3, wobei
dann, wenn ein Bereich des Gesäßbedeckungsabschnitts (C), der sich in der Breitenrichtung (WD) näher an beiden Seiten als das innere Element (200) befindet, gleichmäßig in drei Abschnitte mit einem ersten Bereich (K1), einem zweiten Bereich (K2) und einem dritten Bereich (K3) der Reihe nach von einer Außenseite in der Breitenrichtung (WD) zur Mitte unterteilt wird, für das elastische Element (16a, 16b, 16c) des Bedeckungsabschnitts gilt, dass der Abschnitt des elastischen Elements (16a, 16b, 16c) des Bedeckungsabschnitts, der sich im ersten Bereich (K1) befindet, und der Abschnitt des elastischen Elements (16a, 16b, 16c) des Bedeckungsabschnitts, der sich im dritten Bereich (K3) befindet, jeweils eine höhere Dehnungsrate als der Abschnitt des elastischen Elements (16a, 16b, 16c) des Bedeckungsabschnitts haben, der sich im zweiten Bereich befindet.

5. Wegwerfwindel des Unterhosentyps nach einem der Ansprüche 1 bis 4, wobei
die Anzahl der elastischen Elemente (16a, 16b, 16c) des Bedeckungsabschnitts drei oder vier beträgt.

## Revendications

1. Couche jetable de type culotte comprenant :
un élément externe avant (12F) rectangulaire formant au moins une partie inférieure de torse d'un corps avant et un élément externe arrière (12B) rectangulaire formant au moins une partie inférieure de torse d'un corps arrière indépendamment, l'élément externe avant (12F) et l'élément externe arrière (12B) étant séparés l'un de l'autre au niveau d'une partie intermédiaire de direction avant-arrière (LD) dans la direction avant-arrière (LD) ;
un élément interne (200) comprenant un absorbeur (56) s'étendant dans la direction avant-arrière (LD) depuis l'élément externe avant (12F) jusqu'à l'élément externe arrière (12B), et étant lié à l'élément externe avant (12F) et à l'élément externe arrière (12B) respectivement ; et
une partie d'étanchéité latérale (12A) à laquelle les deux côtés de l'élément externe avant (12F) et les deux côtes de l'élément externe arrière (12B) sont liés pour former une ouverture de taille (WO) et une paire d'ouvertures de jambes (LO) gauche et droite,
l'élément externe arrière (12B) présentant une partie de couverture des fesses (C) s'étendant plus près d'un centre dans la direction avant-arrière (LD) que la partie d'étanchéité latérale (12A), une longueur de direction avant-arrière (LD) de l'élément externe arrière (12B) étant de 1,1 à 1,5 fois une longueur de direction avant-arrière (LD) de la partie d'étanchéité latérale (12A), et la partie de couverture des fesses (C) présentant trois éléments élastiques de partie de couverture (16a, 16b, 16c) allongés ou plus, le long d'une direction de largeur (WD) à intervalles dans la direction avant-arrière (LD) au moins dans une région située plus près des deux côtés dans la direction de largeur (WD) que l'élément interne (200), et la partie de couverture des fesses (C) étant contractée vers un centre dans la direction de largeur (WD) par les éléments élastiques de partie de couverture (16a, 16b, 16c),
dans laquelle l'élément élastique de partie de couverture (16a) situé le plus près de l'ouverture de taille (WO) est disposé au niveau d'une partie d'extrémité de la partie de couverture des fesses (C) sur le côté de l'ouverture de taille (WO), et l'élément élastique de partie de couverture (16c) situé le plus près de l'ouverture de jambe (LO) est disposé au niveau d'une partie d'extrémité de la partie de couverture des fesses (C) sur le côté de l'ouverture de jambe (LO), et
lorsqu'un intervalle central entre l'élément élastique de partie de couverture (16a) situé le plus près de l'ouverture de taille (WO) et l'élément élastique de partie de couverture (16b) situé en deuxième à partir de l'ouverture de taille (WO) est représenté par d_{A}, et une longueur de direction avant-arrière (LD) de la partie de couverture des fesses (C) est représentée par d_{E}, d_{A} > 0,5d_{E} est satisfait.

2. Couche jetable de type culotte selon la revendication 1, dans laquelle
lorsqu'un intervalle central entre des éléments élastiques de partie de couverture (16b, 16c) adjacents situés en deuxième ou ultérieurement à partir de l'ouverture de taille (WO) est représenté par d_{B},
0,05d_{E} < d_{B} < 0,25d_{E} est satisfait.

3. Couche jetable de type culotte selon la revendication 1 ou 2, dans laquelle
lorsqu'un intervalle entre un bord de la partie de couverture des fesses (C) sur le côté de l'ouverture de taille (WO) et un centre d'un élément élastique de partie de couverture (16a) situé le plus près de l'ouverture de taille (WO) est représenté par de, et un intervalle entre un bord (Ce) de la partie de couverture des fesses (C) sur le côté de l'ouverture de jambe (LO) et un centre de l'élément élastique de partie de couverture (16c) situé le plus près de l'ouverture de jambe (LO) est représenté par d_{D},
d_{D} < d_{A} et d_{c} < d_{B} sont satisfaits.

4. Couche jetable de type culotte selon l'une quelconque des revendications 1 à 3, dans laquelle
lorsqu'une région de la partie de couverture des fesses (C) située plus près des deux côtés dans la direction de largeur (WD) que l'élément interne (200) est divisée de manière égale en trois parties d'une première région (K1), d'une deuxième région (K2) et d'une troisième région (K3) séquentiellement à partir d'un extérieur dans la direction de largeur (WD) vers un centre, dans l'élément élastique de partie de couverture (16a, 16b, 16c), cette partie de l'élément élastique de partie de couverture (16a, 16b, 16c) qui est située dans la première région (K1) et cette partie de l'élément élastique de partie de couverture (16a, 16b, 16c) qui est située dans la troisième région (K3) ont chacune un taux d'étirement plus élevé que cette partie de l'élément élastique de partie de couverture (16a, 16b, 16c) qui est située dans la deuxième région.

5. Couche jetable de type culotte selon l'une quelconque des revendications 1 à 4, dans laquelle
le nombre des éléments élastiques de partie de couverture (16a, 16b, 16c) est de trois ou quatre.
